# EUROPEAN PATENT SPECIFICATION

(11) **EP 3 908 276 B1**
(45) Date of publication and mention of the grant of the patent: **11.06.2025**
(21) Application number: 20703633.6
(22) Date of filing: 08.01.2020
(51) Int. Cl.: A61K 31/404, A61P 37/00

(54) **METHODS OF TREATING CONDITIONS RELATED TO THE S1P1 RECEPTOR**
METHODEN ZUR BEHANDLUNG VON S1P1 REZEPTOR BEDINGTEN KRANKHEITEN
MÉTHODES POUR LE TRAITEMENT DE CONDITIONS LIÉES AU RÉCEPTEUR S1P1

(30) Priority: 08.01.2019 US 201962789937 P
(43) Date of publication of application: 17.11.2021
(73) Proprietor: Arena Pharmaceuticals, Inc., New York, NY 10001-2192 (US)
(72) Inventor: NAIK, Snehal, San Diego, California 92130 (US)
(74) Representative: Pfizer
(86) International application number: PCT/US2020/012783
(87) International publication number: WO 2020/146529

(56) References cited:
- WO-A1-2016/112075
- SANDBORN WILLIAM J ET AL: "Efficacy and Safety of Etrasimod in a Phase 2 Randomized Trial of Patients With Ulcerative Colitis", GASTROENTEROLOGY : OFFICIAL PUBLICATION OF THE AMERICAN GASTROENTEROLOGICAL ASSOCIATION, WILLIAMS & WILKINS, US, vol. 158, no. 3, 9 November 2019 (2019-11-09), pages 550 - 561, XP085999260, ISSN: 0016-5085, [retrieved on 20191109], DOI: 10.1053/J.GASTRO.2019.10.035
- ANONYMOUS: "Arena Pharmaceuticals Reports Positive Long-Term Data from the Open-Label Extension of the Phase 2 OASIS Trial Evaluating Etrasimod for Treatment of Ulcerative Colitis", 7 January 2019 (2019-01-07), San Diego, pages 1 - 1, XP055686954, Retrieved from the Internet <URL:http://invest.arenapharm.com/node/19141/pdf> [retrieved on 20200417]
- W J SANDBORN: "569 A randomized, double-blind, placebo-controlled trial of a selective, oral sphingosine 1-phosphate receptor modulator, etrasimod (APD334), in moderate to severe ulcerative colitis: Results from the oasis study", 26TH UNITED EUR GASTROENTEROL WEEK (UEGW), 20 October 2018 (2018-10-20), pages S327 - S328, XP055686935, DOI: 10.1038/ajg.2018.296

## Description

### FIELD

Provided are compounds for use in the treatment of sphingosine 1-phosphate subtype 1 (S1P₁ or SIP1) receptor-associated disorders.

The sphingosine-1-phosphate (S1P) receptors 1-5 constitute a family of G protein-coupled receptors with a seven-transmembrane domain. These receptors, referred to as S1P₁ to S1P₅ (formerly termed endothelial differentiation gene (EDG) receptor-1, -5, -3, -6, and -8, respectively; Chun et al., Pharmacological Reviews, 54:265-269, 2002), are activated *via* binding by sphingosine-1-phosphate, which is produced by the sphingosine kinase-catalyzed phosphorylation of sphingosine. S1P₁, S1P₄, and S1P₅ receptors activate Gi but not Gq, whereas S1P₂ and S1P₃ receptors activate both Gi and Gq. The S1P₃ receptor, but not the S1P₁ receptor, responds to an agonist with an increase in intracellular calcium.

In view of the growing demand for S1P₁ agonists useful in the treatment of S1P₁receptor-associated disorders, the compound (*R*)-2-(7-(4-cyclopentyl-3-(trifluoromethyl)benzyloxy)-1,2,3,4-tetrahydrocyclopenta[b]indol-3-yl)acetic acid (Compound 1, APD334) , or a pharmaceutically acceptable salt, solvate, or hydrate thereof, 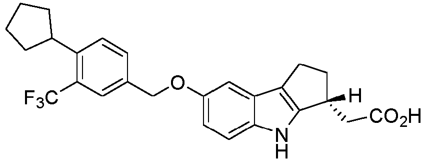 has emerged as an important new compound, see PCT patent application, Serial No. PCT/US2009/004265. Compound 1, or a pharmaceutically acceptable salt, solvate, or hydrate thereof, is an investigational drug candidate intended for the treatment of sphingosine 1-phosphate subtype 1 (S1P₁) receptor-associated disorders.

Many S1P₁ agonists cause side effects, and particularly cardiovascular related adverse events, that require that doctors titrate patients slowly to a maintenance dose. This titration period can take weeks or even a month. The complexity and length of the titration regimen may result in prematurely discontinuing therapy by patients prior to reaching the maintenance dose or to doctors preferring other therapeutic options.

There exists a need for effective treatment with Compound 1, or a pharmaceutically acceptable salt, solvate, or hydrate thereof, in an appropriate patient population. The present disclosure satisfies this need and provides related advantages as well.

Citation of any reference throughout this application is not to be construed as an admission that such reference is prior art to the present application.

In a press release dated 07 January 2019, Arena pharmaceuticals reports positive long-term data from an open-label extension of the Phase 2 OASIS trial evaluating etrasimod for treatment of ulcerative colitis.

In 26th United European Gastroenterology Week (UEGW), 20 October 2018, pages S327-S328, Sandborn discusses a randomized double-blind, placebo-controlled trial of etrasimod in moderate to severe ulcerative colitis.

### SUMMARY

Described herein are results of a clinical study with Compound 1 and the discovery that improvement is not seen in individuals with prior vedolizumab use or a history of pancolitis. Pancolitis (also referred to as universal colitis, total colitis, or pan-ulcerative colitis) is a form of ulcerative colitis that is spread throughout the large intestine.

Provided herein is (*R*)-2-(7-(4-cyclopentyl-3-(trifluoromethyl)benzyloxy)-1,2,3,4-tetrahydrocyclo-penta[b]indol-3-yl)acetic acid (Compound 1), or a pharmaceutically acceptable salt, solvate, or hydrate thereof, for use in a method of treating a sphingosine 1-phosphate subtype 1 (S1P₁) receptor-associated disorder in an individual in need thereof, wherein the S1P₁ receptor-associated disorder is inflammatory bowel disease, the method comprising the steps of: selecting an individual who has not been previously treated with a therapeutically effective amount of an integrin receptor antagonist; and administering to the individual a standard dose of (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof, in an amount equivalent to about 0.5 to about 5.0 mg of Compound 1.

Also provided is (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof, for use in a method of treating a sphingosine 1-phosphate subtype 1 (S1P₁) receptor-associated disorder in an individual in need thereof, wherein in the S1P₁ receptor-associated disorder is inflammatory bowel disease, the method comprising the steps of: selecting an individual who does not have a history of pancolitis; and administering to the individual a standard dose of (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof, in an amount equivalent to about 0.5 to about 5.0 mg of Compound 1.

These and other aspects of the invention disclosed herein will be set forth in greater detail as the patent disclosure proceeds.

### BRIEF DESCRIPTION OF THE DRAWINGS

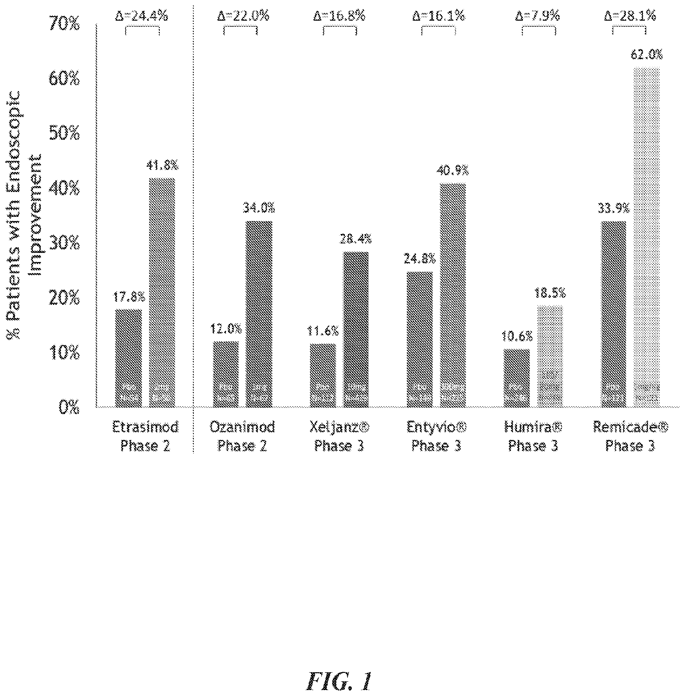

**Figure 1** shows a comparison of percentage of patients with endoscopic improvement, defined as the proportion of patients with a Mayo subscore of 0 or 1, for etrasimod (the L-arginine salt of Compound 1), ozanimod, XELJANZ^{®} (tofacitinib citrate), ENTYVIO^{®} (vedolizumab), SIMPONI^{®} (golimumab), and HUMIRA^{®} (adalimumab), in the trial described in Example 2.

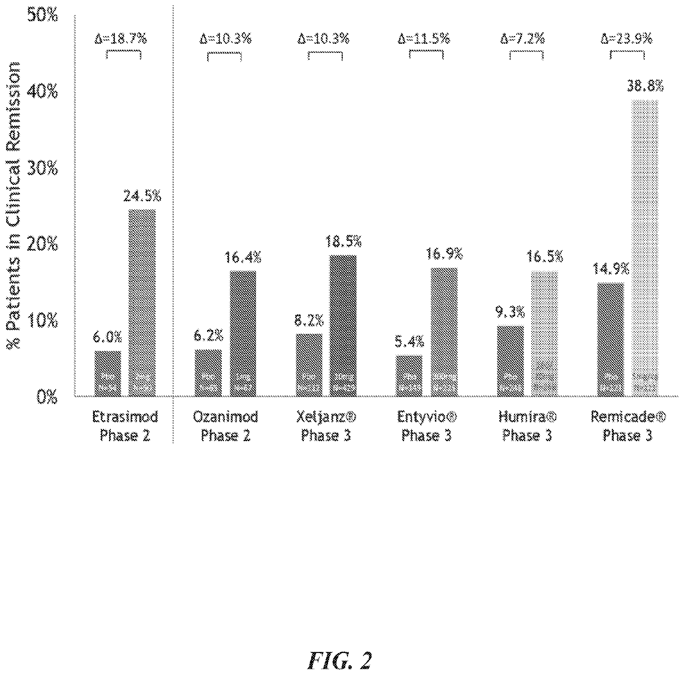

**Figure 2** shows a comparison of percentage of patients in clinical remission, defined as the proportion of patients with total Mayo score ≤ 2 points and no subscore >1, for etrasimod (the L-arginine salt of Compound 1), ozanimod, XELJANZ^{®} (tofacitinib citrate), ENTYVIO^{®} (vedolizumab), SIMPONI^{®} (golimumab), and HUMIRA^{®} (adalimumab), in the trial described in Example 2.

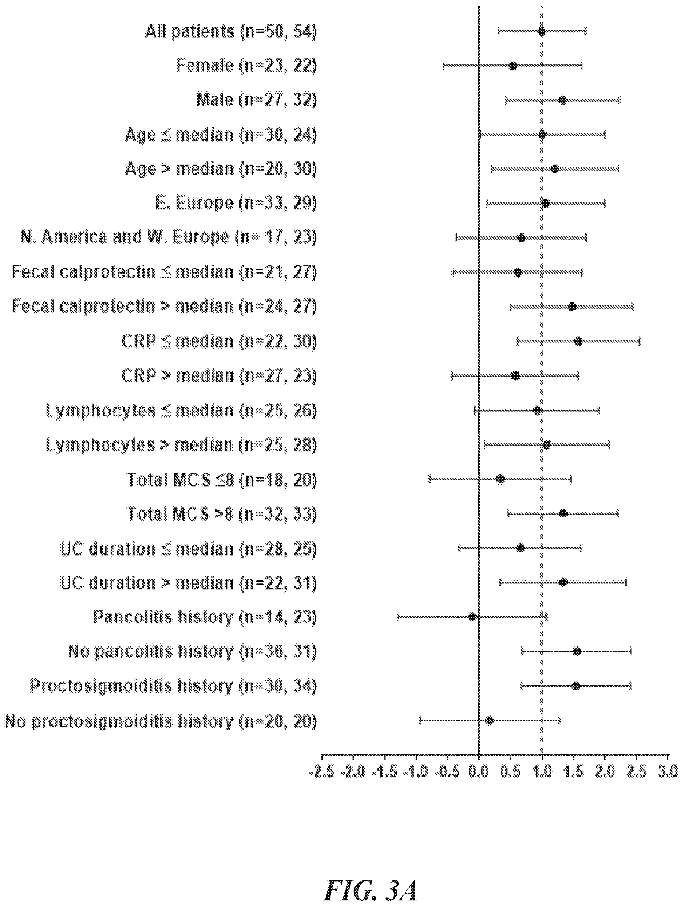

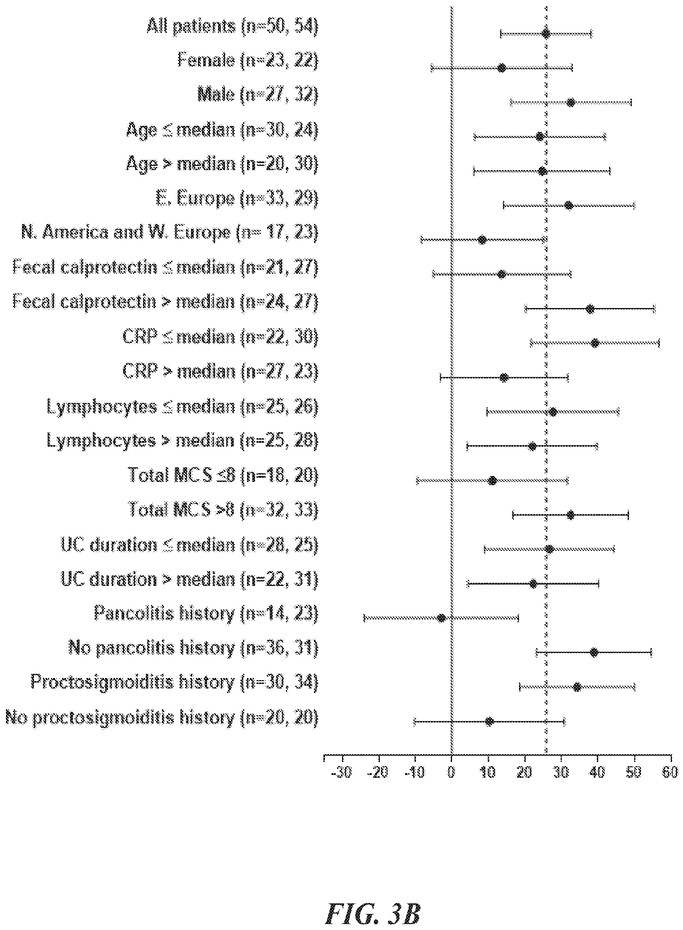

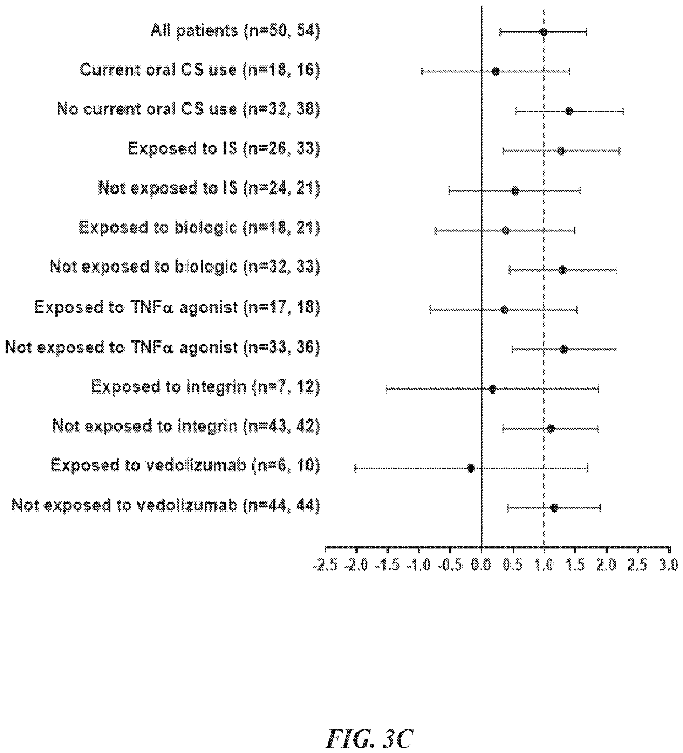

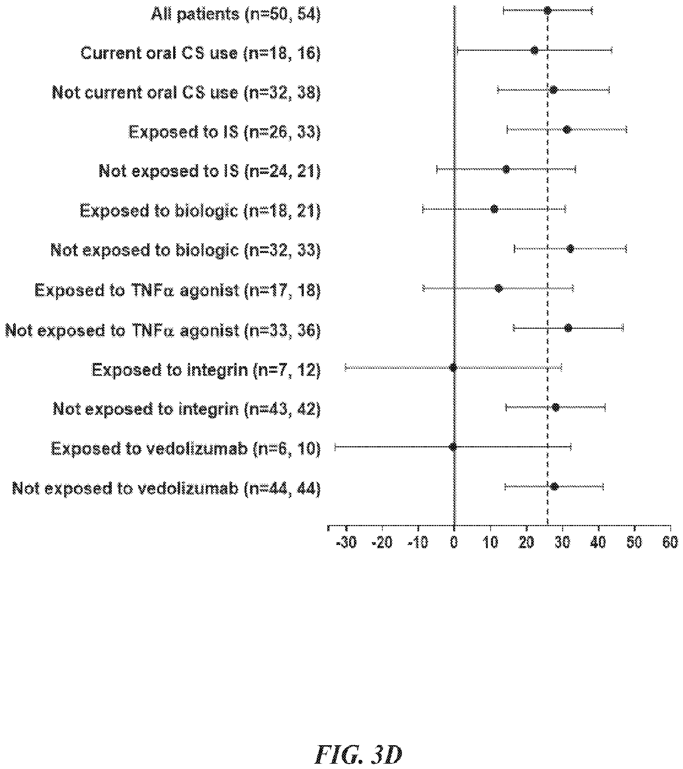

**Figures 3A-3D** show subgroup analyses of placebo-adjusted change (90% confidence interval) at week 12 in the etrasimod 2 mg group in the trial described in Example 2. The improvement in the modified MCS (3A and 3C) and the proportion of patients achieving clinical remission (3B and 3D) are shown for baseline disease characteristics (3A and 3B) and prior or concurrent therapies (3C and 3D). Values less than 0.0 favor placebo, while values greater than 0.0 favor etrasimod (Figures 3A and 3C). Values less than 0 favor placebo, while values greater than 0 favor etrasimod (Figure 3B and 3D). Sample size n = etrasimod 2 mg group, placebo group. CRP = C-reactive protein; CS = corticosteroids; IS = immunosuppressants; MCS = Mayo Clinic score; TNFα = tumor necrosis factor alpha; UC = ulcerative colitis.

### DETAILED DESCRIPTION

As used in the present specification, the following words and phrases are generally intended to have the meanings as set forth below, except to the extent that the context in which they are used indicates otherwise.

**COMPOUND 1:** As used herein, "Compound 1" means (*R*)-2-(7-(4-cyclopentyl-3-(trifluoromethyl)benzyloxy)-1,2,3,4-tetrahydrocyclopenta[*b*]indol-3-yl)acetic acid including crystalline forms thereof. As a non-limiting example, Compound 1 may be present as an anhydrous, non-solvated crystalline form as described in WO 2010/011316. As another non-limiting example, an L-arginine salt of Compound 1 may be present as an anhydrous, non-solvated crystalline form as described in WO 2010/011316 and WO 2011/094008. As another non-limiting example, a calcium salt of Compound 1 may be present as a crystalline form as described in WO 2010/011316.

**ADMINISTERING:** As used herein, "administering" means to provide a compound or other therapy, remedy, or treatment such that an individual internalizes a compound.

**PRESCRIBING:** As used herein, "prescribing" means to order, authorize, or recommend the use of a drug or other therapy, remedy, or treatment. In some embodiments, a health care practitioner can orally advise, recommend, or authorize the use of a compound, dosage regimen or other treatment to an individual. In this case the health care practitioner may or may not provide a prescription for the compound, dosage regimen, or treatment. Further, the health care practitioner may or may not provide the recommended compound or treatment. For example, the health care practitioner can advise the individual where to obtain the compound without providing the compound. In some embodiments, a health care practitioner can provide a prescription for the compound, dosage regimen, or treatment to the individual. For example, a health care practitioner can give a written or oral prescription to an individual. A prescription can be written on paper or on electronic media such as a computer file, for example, on a hand held computer device. For example, a health care practitioner can transform a piece of paper or electronic media with a prescription for a compound, dosage regimen, or treatment. In addition, a prescription can be called in (oral), faxed in (written), or submitted electronically via the internet to a pharmacy or a dispensary. In some embodiments, a sample of the compound or treatment can be given to the individual. As used herein, giving a sample of a compound constitutes an implicit prescription for the compound. Different health care systems around the world use different methods for prescribing and/or administering compounds or treatments and these methods are encompassed by the disclosure.

A prescription can include, for example, an individual's name and/or identifying information such as date of birth. In addition, for example, a prescription can include: the medication name, medication strength, dose, frequency of administration, route of administration, number or amount to be dispensed, number of refills, physician name, physician signature, and the like. Further, for example, a prescription can include a DEA number and/or state number.

A healthcare practitioner can include, for example, a physician, nurse, nurse practitioner, or other related health care professional who can prescribe or administer compounds (drugs) for the treatment of a sphingosine 1-phosphate subtype 1 (S1P₁) receptor-associated disorder. In addition, a healthcare practitioner can include anyone who can recommend, prescribe, administer, or prevent an individual from receiving a compound or drug including, for example, an insurance provider.

**PREVENT, PREVENTING, OR PREVENTION:** As used herein, the term "prevent," "preventing", or "prevention" such as prevention of a sphingosine 1-phosphate subtype 1 (S1P₁) receptor-associated disorder or the occurrence or onset of one or more symptoms associated with the particular disorder and does not necessarily mean the complete prevention of the disorder. For example, the term "prevent," "preventing" and "prevention" means the administration of therapy on a prophylactic or preventative basis to an individual who may ultimately manifest at least one symptom of a disease or condition but who has not yet done so. Such individuals can be identified on the basis of risk factors that are known to correlate with the subsequent occurrence of the disease. Alternatively, prevention therapy can be administered without prior identification of a risk factor, as a prophylactic measure. Delaying the onset of at least one symptom can also be considered prevention or prophylaxis.

**TREAT, TREATING, OR TREATMENT:** As used herein the term "treat," "treating", or "treatment" means the administration of therapy to an individual who already manifests at least one symptom of a disease or condition or who has previously manifested at least one symptom of a disease or condition. For example, "treating" can include alleviating, abating or ameliorating a disease or condition symptoms, preventing additional symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. For example, the term "treating" in reference to a disorder means a reduction in severity of one or more symptoms associated with that particular disorder. Therefore, treating a disorder does not necessarily mean a reduction in severity of all symptoms associated with a disorder and does not necessarily mean a complete reduction in the severity of one or more symptoms associated with a disorder.

**TOLERATE:** As used herein, an individual is said to "tolerate" a dose of a compound if administration of that dose to that individual does not result in an unacceptable adverse event or an unacceptable combination of adverse events. One of skill in the art will appreciate that tolerance is a subjective measure and that what may be tolerable to one individual may not be tolerable to a different individual. For example, one individual may not be able to tolerate headache, whereas a second individual may find headache tolerable but is not able to tolerate vomiting, whereas for a third individual, either headache alone or vomiting alone is tolerable, but the individual is not able to tolerate the combination of headache and vomiting, even if the severity of each is less than when experienced alone.

**ADVERSE EVENT:** As used herein, an "adverse event" is an untoward medical occurrence that is associated with treatment with Compound 1 or a pharmaceutically acceptable salt, solvate, or hydrate thereof. In one embodiment, an adverse event is selected from: leukopenia, constipation, diarrhea, nausea, abdominal pain, neutropenia, vomiting, back pain, and menstrual disorder. In one embodiment, an adverse event is heart block, for example, a first degree atrioventricular heart block. In one embodiment, an adverse event is an acute heart rate reduction. In one embodiment, an adverse event is an abnormal pulmonary function test finding, such as an FEV1 below 80%, FVC. In one embodiment, an adverse event is an abnormal liver function test, such as an elevated ALT & AST>2X ULN. In one embodiment, an adverse event is macular edema.

**IN NEED OF TREATMENT** and **IN NEED THEREOF:** As used herein, "in need of treatment" and "in need thereof' when referring to treatment are used interchangeably to mean a judgment made by a caregiver (*e.g.* physician, nurse, nurse practitioner, *etc.* in the case of humans) that an individual requires or will benefit from treatment. This judgment is made based on a variety of factors that are in the realm of a caregiver's expertise, but that includes the knowledge that the individual is ill, or will become ill, as the result of a disease, condition or disorder that is treatable by the compounds of the invention. Accordingly, the compounds of the invention can be used in a protective or preventive manner; or compounds of the invention can be used to alleviate, inhibit or ameliorate the disease, condition or disorder.

**ACUTE HEART RATE REDUCTION:** As used herein, "acute heart rate reduction" means a heart rate decrease from normal sinus rhythm of, for example, 10 or more beats per minute (bpm), such as less than about 5 bpm, *e.g.,* less than about 4 bpm or less than about 3 bpm or less than 2 bpm, that is maximal within a few hours, for example 1-3 hours, after drug administration, and thereafter the heart rate returns towards the pre-dose value.

**NORMAL SINUS RHYTHM:** As used herein, "normal sinus rhythm" means the sinus rhythm of the individual when not undergoing treatment. The evaluation of normal sinus rhythm is within the ability of a physician. A normal sinus rhythm will generally give rise to a heart rate in the range from 60-100 bpm.

**DOSE:** As used herein, "dose" means a quantity of Compound 1, or a pharmaceutically acceptable salt, solvate, or hydrate thereof, given to the individual for treating or preventing the disease or disorder at one specific time.

**STANDARD DOSE:** As used herein, "standard dose" means the dose of Compound 1, or a pharmaceutically acceptable salt, solvate, or hydrate thereof, that is given to the individual for treating or preventing the disease or disorder. In some embodiments, administration of the standard dose achieves a target reduction in peripheral blood lymphocyte counts, *e.g.,* a reduction in baseline of at least 35%, such as at least 40%, such as at least 45%, such as at least 50%, such as at least 55%, such as at least 60%, such as at least 65%, such as at least 70%. In some embodiments, administration of the standard dose achieves a reduction in baseline of about 35% to about 70%, such as about 40% to about 65%, such as about 50% to about 65%. In some embodiments, administration of the standard dose achieves target peripheral blood lymphocyte counts, *e.g.,* less than 1000 lymphocytes per microliter, such as 400-800 lymphocytes per microliter. The target dose may vary depending on the nature and severity of the disease to be treated.

**MAYO CLINIC SCORE (MCS):** As used herein, "Mayo Clinic Score" or "MCS" means an instrument designed to measure disease activity of ulcerative colitis and consists of up to 4 subscores: stool frequency, rectal bleeding, findings of flexible proctosigmoidoscopy, and physician global assessment with each component ranging from 0 to 3 (0=normal, 1=mild, 2=moderate, 3=severe). Total score therefore ranges from 0 to 12, with a higher score indicating more severe disease. The 6-point Mayo score is based on stool frequency and rectal bleeding PROs collected daily using electronic patient diaries and excludes the findings on endoscopy and the physician's global assessment. The 3-point Mayo score is based on stool frequency, rectal bleeding, and findings on endoscopy and has a total score ranging from 0 to 9. The 2-point Mayo score is based on rectal bleeding and findings on endoscopy and has a total score ranging from 0 to 6. The physician's global assessment acknowledges the three other criteria findings of the MCS, the individual's daily record of abdominal discomfort and general sense of well-being, and other observations, such as physical findings and the individual's performance.

**MILDLY TO MODERATELY ACTIVE ULCERATIVE COLITIS:** As used herein, "mildly to moderately active ulcerative colitis" means ulcerative colitis characterized by a 4-component MCS of 4 to 10.

**MODERATELY TO SEVERELY ACTIVE ULCERATIVE COLITIS:** As used herein, "moderately to severely active ulcerative colitis" means ulcerative colitis characterized by a 3-component MCS of 4 to 9 including an endoscopic subscore of ≥ 2 and a rectal bleeding score of ≥ 1. The 3-component MCS uses 3 of the 4 components of the complete MCS (endoscopic findings, rectal bleeding, and stool frequency).

**CLINICAL REMISSION:** As used herein, "clinical remission" with respect to ulcerative colitis means a 3-component Mayo Clinic score as follows: an endoscopy score (using flexible proctosigmoidoscopy) of 0 or 1, a rectal bleeding score of 0, and a stool frequency score of 0 or 1 with a decrease of ≥ 1 point from baseline subscore.

**CLINICAL RESPONSE:** As used herein, "clinical response" with respect to ulcerative colitis means a reduction in the 3-component Mayo Clinic score of ≥ 2 points and a decrease of ≥ 30% from baseline with an accompanying decrease in rectal bleeding subscore of ≥ 1 or absolute rectal bleeding score of 0 or 1.

**ENDOSCOPIC IMPROVEMENT:** As used herein, "endoscopic improvement" with respect to ulcerative colitis means ulcerative colitis characterized by a Mayo endoscopic subscore (using findings of flexible proctosigmoidoscopy) of ≤ 1 point.

**ENDOSCOPIC REMISSION:** As used herein, "endoscopic remission" with respect to ulcerative colitis means ulcerative colitis characterized by findings from flexible proctosigmoidoscopy subscore of the Mayo Clinic score = 0.

**IMPROVEMENT IN RECTAL BLEEDING:** As used herein, "improvement in rectal bleeding" with respect to ulcerative colitis means a change from baseline < 0.

**HISTOLOGIC HEALING:** As used herein, "histologic healing" with respect to ulcerative colitis means a score of < 3.1 on the Geboes Index.

**IMPROVEMENT IN STOOL FREQUENCY:** As used herein, "improvement in stool frequency" with respect to ulcerative colitis means a change from baseline < 0.

**5-AMINOSALICYLATES:** As used herein, "5-aminosalicylates", means a class of drugs that include, for example, CANASA^{®} (mesalamine), COLAZAL^{®} (balsalazide disodium), ASACOL^{®} (mesalamine), DELZICOL^{®} (mesalamine), and DIPENTUM^{®} (olsalazine).

**IMMUNOSUPPRESSIVES:** As used herein, "immunosuppressives", means a class of drugs that include, for example, AZASAN^{®} (Azathioprine), IMURAN^{®} (Azathioprine), GENGRAF^{®} (Cyclosporine), NEORAL^{®} (Cyclosporine), and SANDIMMUNE^{®} (Cyclosporine).

**GLUCOCORTICOSTEROIDS:** As used herein, "glucocorticosteroids", means a class of drugs that include, for example, UCERIS^{®} (budesonide); DELTASONE^{®} (prednisone), MEDROL^{®} (methylprednisolone), and hydrocortisone.

**TNFα ANTAGONISTS:** As used herein, "TNFα antagonists" or "tumor necrosis factor-a antagonists", means a class of drugs that include, for example, SIMPONI^{®} (golimumab), REMICADE^{®} (infliximab), and HUMIRA^{®} (adalimumab).

**INTEGRIN RECEPTOR ANTAGONISTS:** As used herein, "integrin receptor antagonists" or "integrin antagonists" means a class of drugs that include, for example, ENTYVIO^{®} (vedolizumab).

**PHARMACEUTICAL COMPOSITION:** As used here, "pharmaceutical composition" means a composition comprising at least one active ingredient, such as Compound 1; including but not limited to, salts, solvates, and hydrates of Compound 1, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

**AGONIST:** As used herein, "agonist" means a moiety that interacts with and activates a G-protein-coupled receptor, such as the S1P₁ receptor, such as can thereby initiate a physiological or pharmacological response characteristic of that receptor. For example, an agonist activates an intracellular response upon binding to the receptor, or enhances GTP binding to a membrane. In certain embodiments, an agonist of the invention is an S1P₁ receptor agonist that is capable of facilitating sustained S1P₁ receptor internalization (see *e.g.,* Matloubian et al., Nature, 427, 355, 2004).

**ANTAGONIST:** As used herein, "antagonist" means a moiety that competitively binds to the receptor at the same site as an agonist (for example, the endogenous ligand), but which does not activate the intracellular response initiated by the active form of the receptor and can thereby inhibit the intracellular responses by an agonist or partial agonist. An antagonist does not diminish the baseline intracellular response in the absence of an agonist or partial agonist.

**HYDRATE:** As used herein, "hydrate" means a compound of the invention or a salt thereof, that further includes a stoichiometric or non-stoichiometric amount of water bound by non-covalent intermolecular forces.

**SOLVATE:** As used herein, "solvate" means a compound of the invention or a salt, thereof, that further includes a stoichiometric or non-stoichiometric amount of a solvent bound by non-covalent intermolecular forces. Preferred solvents are volatile, non-toxic, and/or acceptable for administration to humans in trace amounts.

The compounds according to the invention may optionally exist as pharmaceutically acceptable salts including pharmaceutically acceptable acid addition salts prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, p-toluenesulfonic and the like, such as those pharmaceutically acceptable salts listed by Berge et al., Journal of Pharmaceutical Sciences, 66:1-19 (1977).

The acid addition salts may be obtained as the direct products of compound synthesis. In the alternative, the free base may be dissolved in a suitable solvent containing the appropriate acid and the salt isolated by evaporating the solvent or otherwise separating the salt and solvent. The compounds of this invention may form solvates with standard low molecular weight solvents using methods known to the skilled artisan.

It is understood that when the phrase "pharmaceutically acceptable salts, solvates and hydrates" or the phrase "pharmaceutically acceptable salt, solvate, or hydrate" is used when referring to Compound 1, it embraces pharmaceutically acceptable solvates and/or hydrates of Compound 1, pharmaceutically acceptable salts of Compound 1, as well as pharmaceutically acceptable solvates and/or hydrates of pharmaceutically acceptable salts of Compound 1. It is also understood that when the phrase "pharmaceutically acceptable solvates and hydrates" or the phrase "pharmaceutically acceptable solvate or hydrate" is used when referring to Compound 1that are salts, it embraces pharmaceutically acceptable solvates and/or hydrates of such salts.

It will be apparent to those skilled in the art that the dosage forms described herein may comprise, as the active component, either Compound 1 or a pharmaceutically acceptable salt or as a solvate or hydrate thereof. Moreover, various hydrates and solvates of Compound 1 and their salts will find use as intermediates in the manufacture of pharmaceutical compositions. Typical procedures for making and identifying suitable hydrates and solvates, outside those mentioned herein, are well known to those in the art; see for example, pages 202-209 of K.J. Guillory, "Generation of Polymorphs, Hydrates, Solvates, and Amorphous Solids," in: Polymorphism in Pharmaceutical Solids, ed. Harry G. Britain, Vol. 95, Marcel Dekker, Inc., New York, 1999. Accordingly, one aspect of the present disclosure pertains to methods of prescribing and/or administering hydrates and solvates of Compound 1 and/or its pharmaceutical acceptable salts, that can be isolated and characterized by methods known in the art, such as, thermogravimetric analysis (TGA), TGA-mass spectroscopy, TGA-Infrared spectroscopy, powder X-ray diffraction (XRPD), Karl Fisher titration, high resolution X-ray diffraction, and the like. There are several commercial entities that provide quick and efficient services for identifying solvates and hydrates on a routine basis. Example companies offering these services include Wilmington PharmaTech (Wilmington, DE), Avantium Technologies (Amsterdam) and Aptuit (Greenwich, CT).

The present disclosure includes all isotopes of atoms occurring in the present compounds, salts, solvates, and hydrates. Isotopes include those atoms having the same atomic number but different mass numbers. One aspect of the present invention includes every combination of one or more atoms in the present compounds, salts, solvates, and hydrates that is replaced with an atom having the same atomic number but a different mass number. One such example is the replacement of an atom that is the most naturally abundant isotope, such as ¹H or ¹²C, found in one the present compounds, salts, solvates, and hydrates, with a different atom that is not the most naturally abundant isotope, such as ²H or ³H (replacing ¹H), or ¹¹C, ¹³C, or ¹⁴C (replacing ¹²C). When such a replacement has taken place it is commonly referred to as being isotopically-labeled. Isotopic-labeling of the present compounds, salts, solvates, and hydrates can be accomplished using any one of a variety of different synthetic methods know to those of ordinary skill in the art and they are readily credited with understanding the synthetic methods and available reagents needed to conduct such isotopic-labeling. By way of general example, and without limitation, isotopes of hydrogen include ²H (deuterium) and ³H (tritium). Isotopes of carbon include ¹¹C, ¹³C, and ¹⁴C. Isotopes of nitrogen include ¹³N and ¹⁵N. Isotopes of oxygen include ¹⁵O, ¹⁷O, and ¹⁸O. An isotope of fluorine includes ¹⁸F. An isotope of sulfur includes ³⁵S. An isotope of chlorine includes ³⁶Cl. Isotopes of bromine include ⁷⁵Br, ⁷⁶Br, ⁷⁷Br, and ⁸²Br. Isotopes of iodine include ¹²³I, ¹²⁴I, ¹²⁵I, and ¹³¹I. Another aspect of the present invention includes compositions, such as, those prepared during synthesis, preformulation, and the like, and pharmaceutical compositions, such as, those prepared with the intent of using in a mammal for the treatment of one or more of the disorders described herein, comprising one or more of the present compounds, salts, solvates, and hydrates, wherein the naturally occurring distribution of the isotopes in the composition is perturbed. Another aspect of the present invention includes compositions and pharmaceutical compositions comprising the compounds, salts, solvates, and hydrates, as described herein wherein the salt is enriched at one or more positions with an isotope other than the most naturally abundant isotope. Methods are readily available to measure such isotope perturbations or enrichments, such as, mass spectrometry, and for isotopes that are radio-isotopes additional methods are available, such as, radio-detectors used in connection with HPLC or GC.

Compounds of the present invention can be converted to "prodrugs." The term "prodrugs" means compounds that have been modified with specific chemical groups known in the art and that when administered into an individual undergo biotransformation to give the parent compound. Prodrugs can thus be viewed as compounds of the invention containing one or more specialized non-toxic protective groups used in a transient manner to alter or to eliminate a property of the compound. In one general aspect, the "prodrug" approach is utilized to facilitate oral absorption. A thorough discussion is provided in T. Higuchi and V. Stella, Prodrugs as Novel Delivery Systems Vol. 14 of the A.C.S. Symposium Series; and in Bioreversible Carriers in Drug Design, ed. Edward B. Roche, American Pharmaceutical Association and Pergamon Press, 1987.

When an integer is used in a method disclosed herein, the term "about" can be inserted before the integer.

Throughout this specification, unless the context requires otherwise, the word "comprise", or variations such as "comprises" or "comprising" will be understood to imply the inclusion of a stated step or element or integer or group of steps or elements or integers but not the exclusion of any other step or element or integer or group of elements or integers.

Throughout this specification, unless specifically stated otherwise or the context requires otherwise, reference to a single step, composition of matter, group of steps, or group of compositions of matter shall be taken to encompass one and a plurality (*i.e.* one or more) of those steps, compositions of matter, groups of steps, or groups of compositions of matter.

Each embodiment described herein is to be applied *mutatis mutandis* to each and every other embodiment unless specifically stated otherwise.

The present invention(s) is not to be limited in scope by the specific embodiments described herein, which are intended for the purpose of exemplification only. Functionally-equivalent products, compositions, and methods are clearly within the scope of the invention(s), as described herein.

It is appreciated that certain features of the invention(s), which are, for clarity, described in the context of separate embodiments, can also be provided in combination in a single embodiment. Conversely, various features of the invention(s), which are, for brevity, described in the context of a single embodiment, can also be provided separately or in any suitable subcombination. For example, a method that recites prescribing and/or administering Compound 1 or a pharmaceutically acceptable salt, solvate, or hydrate thereof can be separated into two methods; one method reciting prescribing Compound 1 or a pharmaceutically acceptable salt, solvate, or hydrate thereof and the other method reciting administering Compound 1 or a pharmaceutically acceptable salt, solvate, or hydrate thereof. In addition, for example, a method that recites prescribing Compound 1 or a pharmaceutically acceptable salt, solvate, or hydrate thereof and a separate method of the invention reciting administering Compound 1 or a pharmaceutically acceptable salt, solvate, or hydrate thereof can be combined into a single method reciting prescribing and/or administering Compound 1 or a pharmaceutically acceptable salt, solvate, or hydrate thereof.

Also provided herein is (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof, for use in a method of treating ulcerative colitis in an individual in need thereof, the method comprising the steps of: selecting an individual who has not been previously treated with a therapeutically effective amount of vedolizumab; and administering to the individual 2 mg of (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof.

Also provided is (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof, for use in a method of treating ulcerative colitis in an individual in need thereof, comprising the steps of: selecting an individual who does not have a history of pancolitis; and administering to the individual 2 mg of (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically acceptable salt, solvate, or hydrate thereof.

In some embodiments, the individual with a history of pancolitis had pancolitis for about, or greater than about, 1, 2, 3, 4, 5, 6, 7, 8, 9, or 10 years.

In some embodiments, the individual with a history of pancolitis has had pancolitis for about, or greater than about, 1, 2, 3, 4, 5, 6, 7, 8, 9, or 10 years.

In some embodiments, the individual with a history of pancolitis had pancolitis for about, or greater than about, 8 years.

In some embodiments, the individual with a history of pancolitis has had pancolitis for about, or greater than about, 8 years.

In some embodiments, the individual has a baseline lymphocyte count of at least about 800, 825, 850, 875, 900, 925, 950, 975, or 1000 lymphocytes per microliter.

In some embodiments, the individual has been exposed to less than or equal to one biologic agent.

In some embodiments, the individual has not been exposed to any biologic agents.

In some embodiments, the individual has been exposed to a janus kinase (JAK) inhibitor approved for the treatment of ulcerative colitis.

In some embodiments, the biologic agent is selected from golimumab, infliximab, adalimumab, vedolizumab, ustekinumab, and etrolizumab.

In some embodiments, the integrin receptor antagonist is an α4β7 integrin receptor antagonist.

In some embodiments, the integrin receptor antagonist is vedolizumab.

In some embodiments, the therapeutically effective amount of vedolizumab is about, or at least about, 300 mg.

In some embodiments, the integrin receptor antagonist is natalizumab (TYSABRI^{®}).

In some embodiments, the therapeutically effective amount of natalizumab is about, or at least about, 300 mg.

In some embodiments, the integrin receptor antagonist is etrolizumab.

In some embodiments, the integrin receptor antagonist is abrilumab.

In some embodiments, the integrin receptor antagonist is SHP647.

In some embodiments, the individual had an inadequate response with, lost response to, or was intolerant to the integrin receptor antagonist.

In some embodiments, the individual had an inadequate response with, lost response to, or was intolerant to vedolizumab.

In some embodiments, the individual had an inadequate response with, lost response to, or was intolerant to natalizumab.

In some embodiments, the individual had demonstrated, over the previous 3 month period, an inadequate response to, loss of response to, or intolerance of the integrin receptor antagonist. In some embodiments, the individual had demonstrated, over the previous 6 month period, an inadequate response to, loss of response to, or intolerance of the integrin receptor antagonist. In some embodiments, the individual had demonstrated, over the previous 9 month period, an inadequate response to, loss of response to, or intolerance of the integrin receptor antagonist. In some embodiments, the individual had demonstrated, over the previous 1 year period, an inadequate response to, loss of response to, or intolerance of the integrin receptor antagonist. In some embodiments, the individual had demonstrated, over the previous 2 year period, an inadequate response to, loss of response to, or intolerance of the integrin receptor antagonist. In some embodiments, the individual had demonstrated, over the previous 3 year period, an inadequate response to, loss of response to, or intolerance of the integrin receptor antagonist. In some embodiments, the individual had demonstrated, over the previous 4 year period, an inadequate response to, loss of response to, or intolerance of the integrin receptor antagonist. In some embodiments, the individual had demonstrated, over the previous 5 year period, an inadequate response to, loss of response to, or intolerance of the integrin receptor antagonist.

In some embodiments, an individual is selected for treatment based on their baseline lymphocyte count. In some embodiments, the individual is selected for treatment based on a baseline lymphocyte count to increase the likelihood of efficacy. In some embodiments, the individual is selected for treatment based on a baseline lymphocyte count to increase the likelihood of safety. In some embodiments, the individual has a baseline lymphocyte count of 500-1000 lymphocytes per microliter. In some embodiments, the individual has a baseline lymphocyte count of, or of about, at least 400, 450, 500, 550, 600, 625, 650, 675, 700, 725, 750, 775, 800, 825, 850, 875, 900, 925, 950, 975, or 1000 lymphocytes per microliter. In some embodiments, the individual has a baseline lymphocyte count of at least 750 lymphocytes per microliter. In some embodiments, the individual has a baseline lymphocyte count of at least 800 lymphocytes per microliter. In some embodiments, the individual has a baseline lymphocyte count of at least 900 lymphocytes per microliter. In some embodiments, the individual has a baseline lymphocyte count of at least 1000 lymphocytes per microliter.

In some embodiments, an individual is selected for treatment based on the number of biologic agents the individual has been exposed to. In some embodiments, an individual selected for treatment has been exposed to ≤ 1 biologic agents. In some embodiments, an individual selected for treatment has been exposed to ≤ 2 biologic agents. In some embodiments, an individual selected for treatment has not been exposed to ≥1 biologic agent. In some embodiments, an individual selected for treatment has not been exposed to ≥2 biologic agents. In some embodiments, an individual selected for treatment has not been exposed to ≥2 biologic agents plus a janus kinase (JAK) inhibitor approved for the treatment of ulcerative colitis. In some embodiments, an individual selected for treatment has not been exposed to ≥2 biologic agents plus a janus kinase (JAK) inhibitor being investigated for the treatment of ulcerative colitis. In some embodiments, an individual selected for treatment has not been exposed to ≥3 biologic agents.

In some embodiments, the biologic agent is a TNFα antagonist. In some embodiments, the biologic agent is selected from golimumab (SIMPONI), infliximab (REMICADE), and adalimumab (HUMIRA). In some embodiments, the biologic agent is an integrin receptor antagonist. In some embodiments, the biologic agent is vedolizumab (ENTYVIO). In some embodiments, the biologic agent is ustekinumab (STELARA). In some embodiments, the biologic agent is etrolizumab. In some embodiments, the biologic agent is a biosimilar to one of the biologic agents provided herein.

In some embodiments, the JAK inhibitor is selected from tofacitinib (XELJANZ), filgotinib (Galapagos NV), peficitinib (SMYRAF), upadacitinib (RINVOQ), TD-1473 (Theravance Biopharma), or combinations thereof.

In some embodiments, treating comprises inducing and/or maintaining clinical response; improving endoscopic appearance of the mucosa; and/or inducing and/or maintaining clinical remission.

In some embodiments, the standard dose is administered without titration.

In some embodiments, prior to the administering the individual has a 3-component Mayo Clinic Score of at least 6.

In some embodiments, the method results in an improvement of the individual's 3-component Mayo Clinic Score. In some embodiments, the method results in an improvement of the individual's 2-component Mayo Clinic Score. In some embodiments, the method results in an improvement of the individual's Total Mayo Clinic Score.

In some embodiments of the method of treatment of inflammatory bowel disease, *e.g.,* ulcerative colitis, such as moderately to severely active ulcerative colitis, the treatment results in endoscopic improvement, *e.g.,* improving endoscopic appearance of the mucosa.

In some embodiments of the method of treatment of inflammatory bowel disease, *e.g.,* ulcerative colitis, such as moderately to severely active ulcerative colitis, the treatment results in inducing clinical remission. In some embodiments of the method of treatment of inflammatory bowel disease, *e.g.,* ulcerative colitis, such as moderately to severely active ulcerative colitis, the treatment results in maintaining clinical remission. In some embodiments of the method of treatment of inflammatory bowel disease, *e.g.,* ulcerative colitis, such as moderately to severely active ulcerative colitis, the treatment results in inducing and maintaining clinical remission.

In some embodiments of the method of treatment of inflammatory bowel disease, *e.g.,* ulcerative colitis, such as moderately to severely active ulcerative colitis, the treatment results in inducing clinical response. In some embodiments of the method of treatment of inflammatory bowel disease, *e.g.,* ulcerative colitis, such as moderately to severely active ulcerative colitis, the treatment results in maintaining clinical response. In some embodiments of the method of treatment of inflammatory bowel disease, *e.g.,* ulcerative colitis, such as moderately to severely active ulcerative colitis, the treatment results in inducing and maintaining clinical response.

In some embodiments, the treatment reduces a lymphocyte count in the individual by at least 40%. In some embodiments, the treatment reduces a lymphocyte count in the individual by at least 45%, 50%, 55%, 60%, or 65%.

In some embodiments of the method of treatment of inflammatory bowel disease, *e.g.,* ulcerative colitis, such as moderately to severely active ulcerative colitis, the treatment results in corticosteroid-free remission.

In some embodiments of the method of treatment of inflammatory bowel disease, *e.g.,* ulcerative colitis, such as moderately to severely active ulcerative colitis, the treatment results in endoscopic remission.

In some embodiments of the method of treatment of inflammatory bowel disease, *e.g.,* ulcerative colitis, such as moderately to severely active ulcerative colitis, the treatment results in an improvement in rectal bleeding.

In some embodiments of the method of treatment of inflammatory bowel disease, *e.g.,* ulcerative colitis, such as moderately to severely active ulcerative colitis, the treatment results in histologic healing.

In some embodiments of the method of treatment of inflammatory bowel disease, *e.g.,* ulcerative colitis, such as moderately to severely active ulcerative colitis, the treatment results in an improvement in stool frequency.

In some embodiments of the method of treatment of inflammatory bowel disease, *e.g.,* ulcerative colitis, such as moderately to severely active ulcerative colitis, the treatment further comprises monitoring the level of fecal calprotectin.

In some embodiments of the method of treatment of inflammatory bowel disease, *e.g.,* ulcerative colitis, such as moderately to severely active ulcerative colitis, the treatment further comprises monitoring the level of c-reactive protein (CRP).

In some embodiments, treating is reducing a sign and/or symptom of ulcerative colitis. In some embodiments, treating is reducing a sign of ulcerative colitis. In some embodiments, treating is reducing a symptom of ulcerative colitis. In some embodiments, treating is reducing a sign and/or symptom of Crohn's disease. In some embodiments, treating is reducing a sign of Crohn's disease. In some embodiments, treating is reducing a symptom of Crohn's disease.

In some embodiments, treating is inducing and/or maintaining clinical remission. In some embodiments, treating is inducing and maintaining clinical remission. In some embodiments, treating is inducing and/or maintaining clinical remission and/or clinical response. In some embodiments, treating is inducing and maintaining clinical remission and clinical response. In some embodiments, treating is inducing clinical remission and/or clinical response. In some embodiments, treating is maintaining clinical remission and/or clinical response. In some embodiments, treating is inducing clinical remission and clinical response. In some embodiments, treating is maintaining clinical remission and clinical response. In some embodiments, treating is inducing and/or maintaining clinical remission and/or mucosal healing. In some embodiments, treating is inducing and maintaining clinical remission and mucosal healing. In some embodiments, treating is inducing and maintaining mucosal healing. In some embodiments, treating is inducing and maintaining clinical remission. In some embodiments, treating is inducing clinical remission. In some embodiments, treating is inducing mucosal healing. In some embodiments, treating is maintaining clinical remission. In some embodiments, treating is maintaining mucosal healing. In some embodiments, treating is achieving and/or sustaining clinical remission in induction responders. In some embodiments, treating is achieving and sustaining clinical remission in induction responders. In some embodiments, treating is achieving clinical remission in induction responders. In some embodiments, treating is sustaining clinical remission in induction responders. In some embodiments, treating is inducing and/or maintaining clinical response. In some embodiments, treating is inducing and maintaining clinical response. In some embodiments, treating is inducing clinical response. In some embodiments, treating is maintaining clinical response. In some embodiments, treating is inducing endoscopic improvement. In some embodiments, treating is maintaining endoscopic improvement. In some embodiments, treating is achieve endoscopic improvement. In some embodiments, treating is improving endoscopic remission. In some embodiments, treating is maintaining endoscopic remission. In some embodiments, treating is inducing histologic healing. In some embodiments, treating is maintaining histologic healing. In some embodiments, treating is improving stool frequency. In some embodiments, treating is maintaining improvement in stool frequency. In some embodiments, treating is improving endoscopic appearance of the mucosa. In some embodiments, treating is maintaining endoscopic improvement of the mucosa. In some embodiments, treating is improving endoscopic appearance of the mucosa during induction. In some embodiments, treating eliminates the need for corticosteroid use. In some embodiments, treating allows for reduced corticosteroid use. In some embodiments, treating allows for the use of a lower dose of a corticosteroid. In some embodiments, treating is achieving corticosteroid-free remission. In some embodiments, treating is sustaining corticosteroid-free remission. In some embodiments, treating is improving rectal bleeding. In some embodiments, treating is maintaining improvement in rectal bleeding. In some embodiments, treating is improving endoscopic subscore. In some embodiments, treating is maintaining improvement in endoscopic subscore.

In some embodiments, prior to said administering the individual has a 3-component Mayo Clinic Score of at least 6.

In some embodiments, the administering results in an improvement of the individual's 3-component Mayo Clinic Score.

In some embodiments, the administering results in an improvement of the individual's 2-component Mayo Clinic Score.

In some embodiments, the administering results in an improvement of the individual's Total Mayo Clinic Score.

In some embodiments, the administering results in improvement in the endoscopic appearance of the mucosa of the individual.

In some embodiments, the administering results in inducing clinical remission in the individual.

In some embodiments, the administering results in maintaining clinical remission in the individual.

In some embodiments, the administering results in inducing and maintaining clinical remission in the individual.

In some embodiments, the administering results in inducing clinical response in the individual.

In some embodiments, the administering results in maintaining clinical response in the individual.

In some embodiments, the administering results in inducing and maintaining clinical response in the individual.

In some embodiments, ulcerative colitis has been diagnosed using a 2-component Mayo Clinic Score. For example, in some embodiments, ulcerative colitis has been diagnosed using a score ranging from 0 to 9 for rectal bleeding and endoscopic findings. In some embodiments, ulcerative colitis has been diagnosed using a 3-component Mayo Clinic Score. For example, in some embodiments, ulcerative colitis has been diagnosed using a score ranging from 0 to 9 for stool frequency, rectal bleeding, and endoscopic findings. In some embodiments, ulcerative colitis has been diagnosed using a Total Mayo Score. For example, in some embodiments, ulcerative colitis has been diagnosed using a score ranging from 0 to 12 for stool frequency, rectal bleeding, endoscopic findings, and Physicians Global Assessment.

In some embodiments, improvement in ulcerative colitis is measured using a 2-component Mayo Clinic Score. In some embodiments, improvement in ulcerative colitis is measured using a 3-component Mayo Clinic Score. In some embodiments, improvement in ulcerative colitis is measured using a Total Mayo Score. In some embodiments, improvement in ulcerative colitis is measured by clinical remission. In some embodiments, improvement in ulcerative colitis is measured by lymphocyte reduction. In some embodiments, improvement in ulcerative colitis is measured by endoscopic improvement. In some embodiments, improvement in ulcerative colitis is measured by 6-point Mayo Score. For example, in some embodiments, improvement in ulcerative colitis is measured by stool frequency and rectal bleeding. In some embodiments, improvement in ulcerative colitis is statistically significant.

In some embodiments, the individual has had an inadequate response with, lost response to, been intolerant to, or demonstrated dependence on an integrin receptor antagonist for the treatment of an inflammatory bowel disease. In some embodiments, the individual has had an inadequate response with an integrin receptor antagonist for the treatment of an inflammatory bowel disease. In some embodiments, the individual has lost response to an integrin receptor antagonist for the treatment of an inflammatory bowel disease. In some embodiments, the individual was intolerant to an integrin receptor antagonist for the treatment of an inflammatory bowel disease.

In some embodiments, the S1P₁ receptor-associated disorder is ulcerative colitis.

In some embodiments, the S1P₁ receptor-associated disorder is selected from one or more of: ulcerative proctitis, proctosigmoiditis, and left-sided colitis.

In some embodiments, the S1P₁ receptor-associated disorder is proctitis.

In some embodiments, the S1P₁ receptor-associated disorder is ulcerative proctitis.

In some embodiments, the S1P₁ receptor-associated disorder is proctosigmoiditis.

In some embodiments, the S1P₁ receptor-associated disorder is left-sided colitis.

In some embodiments, the S1P₁ receptor-associated disorder is Crohn's disease.

In some embodiments, the S1P₁ receptor-associated disorder is an active skin extra-intestinal manifestation of inflammatory bowel disease. In some embodiments, the S1P₁ receptor-associated disorder is an active skin extra-intestinal manifestation of ulcerative colitis. In some embodiments, the active skin extra-intestinal manifestation is psoriasis. In some embodiments, the active skin extra-intestinal manifestation is erythema nodosum. In some embodiments, the active skin extra-intestinal manifestation is pyoderma gangrenosum.

In some embodiments, the S1P₁ receptor-associated disorder is ulcerative colitis. In some embodiments, the S1P₁ receptor-associated disorder is moderately to severely active ulcerative colitis. In some embodiments, the S1P₁ receptor-associated disorder is moderately active ulcerative colitis. In some embodiments, the S1P₁ receptor-associated disorder is severely active ulcerative colitis. In some embodiments, the S1P₁ receptor-associated disorder is mildly to moderately active ulcerative colitis. In some embodiments, the S1P₁ receptor-associated disorder is mildly active ulcerative colitis.

In some embodiments, the standard dose is in an amount equivalent to 1 mg of Compound 1.

In some embodiments, the standard dose is in an amount equivalent to, or to about, 0.5, 0.75, 1, 1.25, 1.5, 1.75, 2, 2.25, 2.5, 2.75, and 3 mg of Compound 1.

In some embodiments, the standard dose is in an amount equivalent to 1.5 mg of Compound 1.

In some embodiments, the standard dose is in an amount equivalent to 2 mg of Compound 1.

In some embodiments, the standard dose is in an amount equivalent to 2.5 mg of Compound 1.

In some embodiments, the standard dose is in an amount equivalent to 3 mg of Compound 1.

In some embodiments, the standard dose of Compound 1, or a pharmaceutically acceptable salt, hydrate, or solvate thereof is administered once daily to the individual.

In some embodiments, the Compound 1, or a pharmaceutically acceptable salt, hydrate, or solvate thereof, is administered orally.

In some embodiments, the Compound 1, or a pharmaceutically acceptable salt, hydrate, or solvate thereof, is formulated as a capsule or tablet suitable for oral administration.

In some embodiments, the Compound 1, or a pharmaceutically acceptable salt, hydrate, or solvate thereof, is selected from: Compound 1; a calcium salt of Compound 1; and an L-arginine salt of Compound 1. In some embodiments, the Compound 1, or a pharmaceutically acceptable salt, hydrate, or solvate thereof, is an L-arginine salt of Compound 1. In some embodiments, the Compound 1, or a pharmaceutically acceptable salt, hydrate, or solvate thereof, is an anhydrous, non-solvated crystalline form of an L-arginine salt of Compound 1. In some embodiments, the Compound 1, or a pharmaceutically acceptable salt, hydrate, or solvate thereof, is an anhydrous, non-solvated crystalline form of Compound 1.

In some embodiments, Compound 1, or, a pharmaceutically acceptable salt, a hydrate or solvate thereof, is administered as a raw or pure chemical, for example as a powder in capsule formulation.

In some embodiments, Compound 1, or, a pharmaceutically acceptable salt, a hydrate or solvate thereof, is formulated as a pharmaceutical composition further comprising one or more pharmaceutically acceptable carriers.

Pharmaceutical compositions may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tabletting lubricants and disintegrants may be used in tablets and capsules for oral administration. The compounds described herein can be formulated into pharmaceutical compositions using techniques well known to those in the art. Suitable pharmaceutically acceptable carriers, outside those mentioned herein, are known in the art; for example, see Remington, The Science and Practice of Pharmacy, 20th Edition, 2000, Lippincott Williams & Wilkins, (Editors: Gennaro *et al.*)

For oral administration, the pharmaceutical composition may be in the form of, for example, a tablet or capsule. The pharmaceutical composition is preferably made in the form of a dosage unit containing a particular amount of the active ingredient. Examples of such dosage units are capsules, tablets, powders, granules or suspensions, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or encapsulating materials.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may be from 0.5 to about 90 percent of the active compound. However, an artisan would know when amounts outside of this range are necessary. Suitable carriers for powders and tablets include magnesium carbonate, magnesium stearate, talc, sugar, lactose, pectin, dextrin, starch, gelatin, tragacanth, methylcellulose, sodium carboxymethyl cellulose, a low melting wax, cocoa butter, and the like. The term "preparation" includes the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets or capsules. Also, the unit dosage form can be a capsule or tablet itself, or it can be the appropriate number of any of these in packaged form.

Further embodiments include the embodiments disclosed in the following Examples, which is not to be construed as limiting in any way.

### EXAMPLES

### EXAMPLE 1

Formulations composed of immediate-release, hard gelatin capsules containing an L-arginine salt of Compound 1 were prepared as shown in **Table 1.**

**TABLE 1**

| | **Formulation** | | | | |
|---|---|---|---|---|---|
| | **0.1 mg** | **0.35 mg** | **0.5 mg** | **1 mg** | **2 mg** |
| L-arginine salt of Compound 1 (mg/capsule) | 0.14 | 0.48 | 0.69 | 1.38 | 2.76 |
| Empty capsule weight (mg)* | 38.0 | 61.0 | 61.0 | 61.0 | 61.0 |
| Total capsule target weight (mg)** | 38.14 | 61.48 | 61.69 | 62.38 | 63.76 |

| | | | | | |
|---|---|---|---|---|---|
| *Approximate weight. Based on capsule specification **Theoretical total weight calculated by combining fill and empty capsule weights together | | | | | |

Placebo formulations composed of hard gelatin capsules containing microcrystalline cellulose was also prepared as shown in **Table 2.**

**TABLE 2**

| | **Placebo for 0.1 mg** | **Placebo for 0.35 mg and 1 mg** | **Placebo for 0.5 mg, 1 mg, and 2 mg** |
|---|---|---|---|
| Microcrystalline cellulose - Avicel PH102 (mg/capsule)* | 0.0 | 0.0 | 1.0* |
| Empty capsule weight (mg) ** | 38.0 | 61.0 | 61.0 |
| Total capsule target weight (mg)*** | 38.0 | 61.0 | 62.0 |

| | | | |
|---|---|---|---|
| *Approximate weight ± 15% **Approximate weight. Based on capsule specification ***Theoretical total weight calculated by combining fill and empty capsule weights together | | | |

### EXAMPLE 2

A randomized, double-blind, placebo-controlled, parallel-group, dose-ranging study to assess safety and efficacy of two orally administered doses (1 mg and 2 mg) of Compound 1 in patients with ulcerative colitis was conducted. The table below provides a summary of demographic data by treatment group.

| | | **Placebo (n=54)** | **L-arginine salt of Compound 1 1 mg (n=52)** | **L-arginine salt of Compound 1 2 mg (n=50)** |
|---|---|---|---|---|
| Mean Age, Years | | 44.8 | 43.2 | 40.4 |
| Sex, n (%) | Male | 32 (59.3) | 30 (57.7) | 27 (54.0) |
| | Female | 22 (40.7) | 22 (42.3) | 23 (46.0) |
| Race, n (%) | White | 51 (94.4) | 48 (92.3) | 49 (98.0) |
| | Non-white | 3 (5.6) | 4 (7.7) | 1 (2.0) |
| Weight, kg | | 75.76 | 73.68 | 70.37 |
| Duration of UC, years (mean ± SD) | | 8.6 ± 7.16 | 7.0 ± 6.11 | 6.2 ± 4.69 |
| Mean Total Mayo Clinic Score | | 8.7 | 8.8 | 8.9 |
| 3-Component Mayo Score (rectal bleeding, stool frequency, endoscopy) | | 6.5 | 6.5 | 6.6 |
| Concomitant Medication Use, n (%) | Oral Corticosteroids | 16 (29.6) | 13 (25.0) | 18 (36.0) |
| Previous Medication Use, n (%) | Aminosalicylate | 53 (98.1) | 49 (94.2) | 46 (92.0) |
| | TNF-antagonist | 18 (33.3) | 15 (28.8) | 17 (34.0) |
| | Integrin antagonist | 12 (22.2) | 4 (7.7) | 7 (14.0) |
| | Immunosuppressive agent | 33 (61.1) | 17 (32.7) | 26 (52.0) |

| | | **Placebo** | **L-arginine salt of Compound 1** | **L-arginine salt of Compound 1** |
|---|---|---|---|---|
| Received Study Treatment | | 54 | 52 | 50 |
| Completed, n (%) | | 48 (88.9) | 47 (90.4) | 46 (92.0) |
| Early Discontinuation, n (%) | | 6 (11.1) | 5 (9.6) | 4 (8.0) |

Patients were randomized into a double-blind, placebo-controlled study to receive once daily (q.d.) doses of the L-arginine salt of Compound 1 (1 mg or 2 mg) or matching placebo in a 1:1:1 ratio for 12 weeks. The trial enrolled 156 patients with moderate to severe ulcerative colitis (3-component Mayo score of 4-9 that includes endoscopic subscore ≥2, rectal bleeding score ≥1).

The treatment formulation was composed of immediate-release, hard gelatin capsules containing L-arginine salt of Compound 1. The placebo was composed of hard gelatin capsules containing microcrystalline cellulose.

The patients had demonstrated, over the previous 5 year period, an inadequate response to, loss of response to, or intolerance of at least one of the following agents:
Oral 5-aminosalicylates (5-ASAs) (e.g., mesalamines);
Corticosteroids wherein the patient exhibited signs and symptoms of persistently active disease despite a history of at least one 4-week induction regimen that included a dose equivalent to prednisone 30 mg daily; or 2 failed attempts to taper corticosteroids to below a dose equivalent to prednisone 10 mg daily; or a history of intolerance of corticosteroids (including, but not limited to Cushing's syndrome, osteopenia/osteoporosis, hyperglycemia, insomnia, and infection);
Immunosuppressives, wherein the patient exhibited signs and symptoms of persistently active disease despite a history of at least one 8-week regimen of oral azathioprine (≥ 1.5 mg/kg) or 6-mercaptopurine mg/kg (≥ 0.75 mg/kg); or a history of intolerance of at least one of these immunosuppressive (including, but not limited to nausea/vomiting, abdominal pain, pancreatitis, LFT abnormalities, lymphopenia, TPMT genetic mutation, infection);
TNFα antagonists, wherein the patient exhibited signs and symptoms of persistently active disease despite a history of completing an induction regimen with at least one of the following:
   infliximab, adalimumab, or golimumab at doses per the current labeling and/or institutional standard of care; or recurrence of symptoms during maintenance dosing with infliximab, adalimumab, or golimumab following prior clinical benefit (discontinuation despite clinical benefit does not qualify); or a history of intolerance to infliximab, adalimumab, or golimumab (including, but not limited to infusion- or injection- related reaction, demyelination, congestive heart failure, infection); or
Integrin antagonists, wherein the patient exhibited recurrence of symptoms during maintenance dosing with vedolizumab following prior clinical benefit (discontinuation despite clinical benefit does qualify); or a history of intolerance to vedolizumab (including, but not limited to infusion related reaction).

Patients were instructed to take their capsule on an empty stomach (overnight fast of approximately 8 hours) and to avoid eating for approximately 1 hour after dosing.

The primary objective of this proof-of-concept study was to determine the effect of treatment with the L-arginine salt of Compound 1 in changing 3-component Mayo Clinic score (score ranging from 0 to 9, including stool frequency, rectal bleeding and findings on endoscopy) at 12 weeks.

Secondary endpoints were the proportion of patients who achieve endoscopic improvement at Week 12; change in 2-component Mayo Score (score ranging from 0 to 6, including rectal bleeding and findings on endoscopy) at Week 12; and change in Total Mayo Score (score ranging from 0 to 12, including stool frequency, rectal bleeding, findings on endoscopy, and physician global assessment) at Week 12.

Exploratory endpoints included change from baseline in lymphocyte counts at Weeks 1, 2, 4, 8, and 12; proportion of patients achieving clinical remission at Week 12; and proportion of patients who achieve clinical response at Week 12.

ANCOVA model, adjusted for current oral corticosteroid use, prior exposure to TNF-alpha antagonists, and baseline value, was used to estimate changes in Mayo Clinic Score. Mantel-Haenszel method (estimated treatment difference by adjusting current oral corticosteroid use and prior exposure to TNF-alpha antagonists) used to estimate proportion difference for dichotomous parameters. Missing individual Mayo subscores impacting efficacy measures were imputed using multiple imputation methodology with observed case analysis for sensitivity. Statistical testing was pre-specified as one-sided with p < 0.025 reflecting conventional statistical significance. Hierarchical closed testing procedure for primary and secondary endpoints at 0.05 alpha level was used.

Patients receiving high dose (2 mg) of the L-arginine salt of Compound 1 achieved the primary and all secondary endpoints with statistical significance.

Relative to placebo, there was a 0.99 improvement in a 3-component Partial Mayo Score (PMS; score ranging from 0 to 9 including stool frequency, rectal bleeding and findings on endoscopy) with the L-arginine salt of Compound 1 at 2 mg at week 12, which was statistically significant (p=0.009). In the low dose (1 mg) group, there was a 0.43 improvement in PMS at week 12 relative to placebo, which was not statistically significant (p=0.146).

Significantly more patients in the L-arginine salt of Compound 1 (2 mg) group achieved endoscopic improvement compared with placebo (41.8% vs. 17.8%, p=0.003). For the 1 mg group, 22.5% of the patients achieved endoscopic improvement (p=0.306).

Relative to placebo, there was a 0.84 improvement in a 2-component Mayo Score (score ranging from 0 to 6, including rectal bleeding and findings on endoscopy) at Week 12, which was statistically significant (p=0.002). In the low dose (1 mg) group, there was a 0.39 improvement relative to placebo, which was not statistically significant (p=0.086).

Relative to placebo, there was a 1.27 improvement in Total Mayo Score (score ranging from 0 to 12, including stool frequency, rectal bleeding, findings on endoscopy, and physician global assessment) at Week 12, (p=0.010) for the 2 mg group. In the low dose (1 mg) group, there was a 0.60 improvement relative to placebo, which was not statistically significant (p=0.128).

In exploratory analyses, the proportion of patients achieving clinical remission, defined by the 3-component Mayo Score, was 33.0% in the L-arginine salt of Compound 1 (2 mg) group compared to 8.1% for placebo group (p < 0.001). For the 1 mg group, 16.0% of the patients achieved clinical remission (p = 0.136)

Remission defined by the 4-component Total Mayo Score was 24.5% and 6.0% for L-arginine salt of Compound 1 and placebo, respectively (p=0.004). Remission for the 1 mg group was 15.4% (p=0.077).

Relative to placebo, there was a 57% reduction in lymphocytes, (p<0.001) for the 2 mg group and a 37% reduction for the 1 mg group, each at 12 weeks.

**Figure 1** shows a comparison of percentage of patients with endoscopic improvement for various treatments for ulcerative colitis. **Figure 2** shows a comparison of percentage of patients in clinical remission, which is defined as the proportion of patients with total Mayo score ≤ 2 points and no subscore >1 for various treatments for ulcerative colitis (remission differed across the studies and the comparison did not result from direct head-to-head studies).

Overall, the results of pre-specified subgroup analyses (including baseline disease characteristics and current and previous treatment) for improvement in modified MCS were similar to those for the primary analysis. The exceptions were prior vedolizumab use and history of pancolitis **(****Figures 3A-3D****).**

The L-arginine salt of Compound 1 was well tolerated and there were fewer serious adverse events (SAEs) compared to placebo (0% in 2 mg; 5.8% in 1 mg; and 11.1% in placebo).

| | **Placebo (n=54)** | **L-arginine salt of Compound 1 1 mg (n=52)** | **L-arginine salt of Compound 1 2 mg (n=50)** |
|---|---|---|---|
| Number (%) of Patients with any TEAE | 27 (50.0) | 31 (59.6) | 28 (56.0) |
| Number (%) of Patients with TEAE Leading to Discontinuation of Study Drug | 0 | 3 (5.8) | 4 (8.0) |
| Number (%) of Patients with Serious TEAE | 6 (11.1) | 3 (5.8) | 0 |
| Number (%) of Deaths of Any Reason | 0 | 0 | 0 |

Impact on heart rate and AV conduction was low throughout the study with no discontinuations from study related to bradycardia or AV block. There were no increases in liver function tests (LFTs) compared to placebo and no reports of macular edema or pulmonary function test abnormalities.

With regard to possible cardiac events, hourly ECGs on Day 1 in both the 1 mg and 2 mg groups showed a mild decrease in heart rate with no mean change in heart rate ≥ 10 bpm in either group at any timepoint. After Day 1, the mean decrease in heart rate from baseline did not exceed 6 bpm in either dose group through 12 weeks. No serious adverse events related to heart rate changes or AV block were recorded.

There were no increases in liver function tests compared to placebo; no reports of macular edema; and no reports of abnormal pulmonary function tests.

The adverse events in infections and infestations were assessed as mild or moderate by investigators. No severe or life-threatening infection occurred. The majority of the adverse events were upper respiratory tract infections.

### EXAMPLE 3

A randomized, double-blind, placebo-controlled 12-week study to assess the safety and efficacy of Compound 1 (2 mg) or placebo in patients with moderately to severely active ulcerative colitis (UC) will be conducted. The efficacy of Compound 1 on clinical remission, clinical response, symptomatic response and remission, endoscopic changes, and mucosal healing when administered for 12 weeks will be assessed. Other objectives include the evaluation of Compound 1 pharmacokinetics (PK) and the effect of Compound 1 on health-related subject-reported outcomes and biomarkers.

Inclusion criteria will including the following:
1. Men or women 16 to 80 years of age, inclusive
2. Ability to provide written informed consent or assent (parent or legal guardian must provide consent for a subject < 18 years of age who has assented to participate in the study or as required per local regulations) and to be compliant with the schedule of protocol assessments
3. Diagnosed with UC ≥ 3 months prior to screening. The diagnosis of UC will be confirmed by endoscopic and histologic evidence.
4. Active UC confirmed by endoscopy with ≥ 10 cm rectal involvement. Inclusion of subjects with proctitis only at baseline will be capped at 15% of the total subjects enrolled.
5. Moderately to severely active UC defined as modified Mayo score (MMS) of 4 to 9, including an endoscopic score (ES) of ≥ 2 and rectal bleeding (RB) score ≥ 1
6. Received a surveillance colonoscopy within 12 months before baseline to rule out dysplasia in subjects with pancolitis > 8 years duration or subjects with left-sided colitis > 12 years duration. Subjects without a surveillance colonoscopy within the prior 12 months will have a colonoscopy at screening (i.e., in place of screening proctosigmoidoscopy). Any adenomatous polyps will be removed prior to first dose of study treatment.
7. Demonstrated an inadequate response to, loss of response to, or intolerance to at least one of the following therapies as defined below:
   Conventional therapy:
      a. Oral 5 aminosalicylic acid (5 ASA) compounds
      b. Corticosteroids
      c. Thiopurines
   Biologic therapy or JAK inhibitor therapy:
      a. Antitumor necrosis factor alpha (TNFα) antibodies (e.g., infliximab, adalimumab, golimumab, or biosimilars)
      b. Anti-integrin antibodies (e.g., vedolizumab)
      c. JAK inhibitors (e.g., tofacitinib)
8. Subjects are permitted to be receiving a therapeutic dose of the following drugs:
   - Oral 5 ASA compounds provided the dose has been stable for ≥ 2 weeks immediately prior to randomization
   - Oral corticosteroid therapy (prednisone at a stable dose ≤ 20 mg/day, budesonide at a stable dose ≤ 9 mg/day, or equivalent steroid) provided the dose has been stable for the 4 weeks immediately prior to the screening endoscopy assessment
   - Immunosuppressive agents such as oral azathioprine or 6 mercaptopurine must be discontinued ≥ 2 weeks prior to randomization
   - Probiotics (e.g., Culturelle^{®}, Saccharomyces boulardii) provided the dose has been stable for the 2 weeks immediately prior to randomization
   - Antidiarrheals (e.g., loperamide, diphenoxylate with atropine) for control of chronic diarrhea If oral aminosalicylates or corticosteroids have been recently discontinued, they must have been stopped for at least 2 weeks prior to the endoscopy used for the baseline MMS.
9. Vital signs at screening and prerandomization taken in the sitting position: heart rate ≥ 50 bpm, systolic blood pressure (BP) ≥ 90 mm Hg, and diastolic BP ≥ 55 mm Hg
10. Screening and prerandomization 12 lead electrocardiogram (ECG) showing no clinically significant abnormalities with a PR interval ≤ 200 ms, Fridericia's corrected QT interval (QTcF) < 450 ms (men) or QTcF < 470 ms (women)
11. Adequate hematological function defined by white blood cell count ≥ 3.5 × 109/L with absolute neutrophil count ≥ 1.5 × 109/L, lymphocyte count ≥ 0.8 × 109/L, platelet count ≥ 100 × 109/L, and hemoglobin ≥ 8 g/dL
12. Adequate hepatic function defined by a total bilirubin level ≤ 1.5 x the upper limit of normal (ULN) range and aspartate aminotransferase (AST) and alanine aminotransferase (ALT) levels ≤ 3.0 × ULN. Subjects with an isolated total bilirubin and normal AST and ALT diagnosed with Gilbert's syndrome may participate
13. Adequate renal function defined by an estimated glomerular filtration rate ≥ 30 mL/min/1.73 m2 by the CKD-EPI equation at screening
14. Eligible women of childbearing potential must be:
   a. Nonpregnant
   b. Not breastfeeding
15. Both men and women subjects agree to use a highly effective method of birth control throughout the entire study period

Exclusion criteria will including the following:
1. Severe extensive colitis as evidenced by:
   - Physician judgment that the subject is likely to require hospitalization for medical care or surgical intervention of any kind for UC (e.g., colectomy) within 12 weeks of baseline
   - Current evidence of fulminant colitis, toxic megacolon or recent history (within last 6 months) of toxic megacolon, or bowel perforation
   - Previous total or partial colectomy
2. Diagnosis of Crohn's disease or indeterminate colitis or the presence or history of a fistula consistent with Crohn's disease
3. Diagnosis of microscopic colitis, ischemic colitis, or infectious colitis
4. Hospitalization for exacerbation of UC requiring intravenous (IV) steroids within 12 weeks of screening (a single dose of IV steroids given is acceptable)
5. Positive assay or stool culture for pathogens or positive test for Clostridium difficile toxin at screening
6. Pregnancy, lactation, or a positive serum β hCG measured during screening
7. Clinically relevant hematologic, hepatic, neurological, pulmonary, ophthalmological, endocrine, metabolic, psychiatric or other major systemic disease making implementation of the protocol or interpretation of the study difficult or would put the subject at risk
8. Recent history (within 2 months of the screening visit) of cardiovascular disease, including myocardial infarction or unstable angina
9. Any history of the following, unless treated with an implanted pacemaker or an implanted cardioverter-defibrillator with pacing:
   - History or presence of symptomatic bradycardia
   - History of sick sinus syndrome or neurocardiogenic syncope
   - Second or third degree AV block
   - Periods of asystole > 3 seconds
10. Forced expiratory volume at 1 second (FEV1) or forced vital capacity (FVC) < 70% of predicted values and FEV1/FVC ratio < 0.70 at screening
11. Uncontrolled diabetes as determined by hemoglobin A1c (HbA1c) > 9% at screening, or subjects with diabetes with significant comorbid conditions such as retinopathy
12. History of macular edema or retinopathy
13. Current or past history of active tuberculosis (TB), history of untreated latent TB infection, or test positive for latent TB infection at screening.
14. Known active bacterial, viral, fungal, mycobacterial infection, or other infection or any major episode of infection that required hospitalization or treatment with IV antibiotics within 30 days of screening or during screening or oral antibiotics within 14 days prior to screening. Fungal infection of nail beds is allowed
15. Have HIV/acquired immune deficiency syndrome or test positive for HIV antibodies at screening
16. Have acute or chronic hepatitis B infection or test positive for hepatitis B virus (HBV) at screening
17. Have current hepatitis C infection or test positive for hepatitis C virus (HCV) at screening as defined by positive for hepatitis C antibody and detectable HCV RNA
18. History of an opportunistic infection (e.g., pneumocystis carinii, cryptococcal meningitis, progressive multifocal leukoencephalopathy) or serious bacterial, viral, or fungal infections (e.g., disseminated herpes simplex, disseminated herpes zoster) and requiring IV medication(s) ≤ 3 weeks prior to randomization
19. History of or currently active primary or secondary immunodeficiency
20. History of cancer within the last five years, including solid tumors and hematological malignancies (except basal cell and in situ squamous cell carcinomas of the skin that have been excised and resolved) or colonic mucosal dysplasia
21. History of lymphoproliferative disorder, lymphoma, leukemia, myeloproliferative disorder, or multiple myeloma
22. History of alcohol or drug abuse within one year prior to randomization
23. Prior treatment with sphingosine 1 phosphate receptor modulators
24. Treatment with a biologic agent within 8 weeks or five elimination half-lives, whichever is shorter
25. Treatment with an investigational therapy within three months prior to randomization
26. Treatment failure with ≥ 3 biologic agents or ≥ 2 biologics plus a JAK inhibitor approved for treatment of UC
27. Treatment with topical rectal 5 ASA, short chain fatty acid enemas, or steroids within two weeks of screening or during screening
28. Treatment with cyclosporine, tacrolimus, sirolimus, methotrexate, or mycophenolate mofetil within 16 weeks of screening
29. Receipt of a live vaccine within 4 weeks prior to randomization
30. Previous treatment with natalizumab
31. Previous treatment with lymphocyte-depleting therapies
32. Previous treatment with D penicillamine, leflunomide, or thalidomide
33. Treatment with IV immune globulin or plasmapheresis within three months prior to randomization
34. Chronic use of therapies that moderately/strongly inhibit/induce cytochrome P450 (CYP) 2C8 and 2C9 metabolism and inhibitors of UGT1A7 within four weeks prior to randomization

Efficacy endpoints will evaluate Compound 1 versus placebo in:
- The proportion of subjects in clinical remission at Week 12
- The proportion of subjects achieving endoscopic improvement at Week 12
- The proportion of subjects with mucosal healing at Week 12
- The proportion of subjects with a clinical response at Week 12
- The proportion of subjects with endoscopic improvement at Week 12 \
- The proportion of subjects with endoscopic normalization at Week 12
- The proportions of subjects with clinical response of RB and SF symptom outcomes at each of the following time points: Weeks 2, 4, 8, and 12
- The proportions of subjects with clinical remission of RB and SF symptom outcomes at each of the following time points: Weeks 2, 4, 8, and 12
- The proportion of subjects with remission and response using total Mayo Clinic score at Week 12
- The proportion of subjects with histologic remission at Week 12 (as defined by the Geboes, Robarts, and Nancy histopathology scores)
- The proportion of subjects with histologic improvement at Week 12 (as defined by the Geboes, Robarts, and Nancy histopathology scores)
- The proportion of subjects with improvement in extraintestinal manifestations (EIMs) at Weeks 12 in subjects with EIMs at baseline
- Scores and change from baseline to Week 12 in the following:
   - inflammatory Bowel Disease Questionnaire (IBDQ) total score
   - Ulcerative Colitis Patient-Reported Outcomes Signs and Symptoms (UC-PRO-SS)
   - Medical Outcomes Study 36-Item Short Form Health Survey (SF-36), version 2, physical and mental component and domain scores
   - Work Productivity and Activity Impairment Questionnaire-Ulcerative Colitis (WPAI-UC)
   - Urgency Numeric Rating Scale (NRS)
   - Abdominal pain NRS
- Proportion of subjects with UC-related hospitalizations
- Proportion of subjects requiring UC-related surgeries, including colectomy
- Plasma concentrations of Compound 1, M3, and other metabolite(s) of interest (if warranted) will be assessed from samples collected prior to dosing and 4 hours (± 15 minutes) post dose (after 12-lead ECG) on Week 0/Day 1
- Plasma concentrations of Compound 1, M3, and other metabolite(s) of interest (if warranted) will be assessed from samples collected prior to dosing (trough) at Weeks 2, 4, 8, and 12
- Change from baseline in level of fecal calprotectin at Weeks 4, 8, and 12
- Change from baseline in level of high-sensitivity C-reactive protein (hs-CRP) at Weeks 2, 4, 8, and 12
- Change and percentage change from baseline in lymphocyte counts at Weeks 2, 4, 8, and 12
- Incidence and severity of adverse events
- Incidence and severity of laboratory abnormalities, and change from baseline in laboratory values (to include hematology, serum chemistry, coagulation, and urinalysis)
- Incidence of clinically significant vital sign abnormalities and changes from baseline

Evaluations will be performed at weeks 2, 4, 8, and 12. Eligible participants will be given the opportunity to enter an open label extension study (OLE) with Compound 1.

### EXAMPLE 4

A randomized, double-blind, placebo-controlled 52-week study to assess the safety and efficacy of Compound 1 (2 mg) or placebo in patients with moderately to severely active ulcerative colitis (UC) will be conducted. The efficacy of Compound 1 on clinical remission, clinical response, symptomatic response and remission, endoscopic changes, and mucosal healing when administered for 52 weeks will be assessed. Other objectives include the evaluation of Compound 1 pharmacokinetics (PK) and the effect of Compound 1 on health-related subject-reported outcomes and biomarkers.

Inclusion and exclusion criteria will be similar to those described in Example 3.

Efficacy endpoints will evaluate Compound 1 versus placebo in:
- The proportion of subjects achieving endoscopic improvement at Week 52
- The proportion of subjects achieving endoscopic improvement at Week 12
- Proportion of subjects, who had not been receiving corticosteroids for ≥ 12 weeks prior to the end of the study (Week 52), with clinical remission at Week 52
- Proportion of subjects with mucosal healing at Week 52
- Proportion of subjects with mucosal healing at Week 12
- Proportion of subjects achieving clinical remission at both Weeks 12 and 52
- Proportion of subjects with a clinical response at Week 52
- Proportion of subjects with a clinical response at Week 12
- Proportion of subjects with endoscopic normalization at Week 52
- Proportion of subjects with endoscopic normalization at Week 12
- Proportion of subjects with symptomatic remission at Week 52
- Proportion of subjects with symptomatic remission at Week 12
- Proportion of subjects with non-invasive clinical response at Weeks 12, 16, 20, 24, 32, 40, 48, and 52
- Proportion of subjects with symptomatic response at Weeks 12, 16, 20, 24, 32, 40, 48, and 52
- Proportion of subjects with remission at Week 52 and corticosteroid-free since Weeks 16, 24, 32, 40, and 48
- Proportion of subjects with clinical remission at Week 52 among subjects in clinical response at Week 12
- Proportion of subjects achieving clinical response at both Weeks 12 and 52
- Proportion of subjects achieving mucosal healing at both Weeks 12 and 52
- Proportion of subjects achieving endoscopic normalization at both Weeks 12 and 52
- Proportions of subjects with clinical response of RB and SF symptom outcomes at Weeks 2, 4, 8, 12, 16, 20, 24, 32, 40, 48, and 52
- Proportions of subjects with clinical remission of RB and SF symptom outcomes at Weeks 2, 4, 8, 12, 16, 20, 24, 32, 40, 48, and 52
- Proportion of subjects with remission and response using total Mayo Clinic score at Week 12
- Proportion of subjects with remission and response using total Mayo Clinic score at Week 52
- Proportion of subjects with histologic improvement at Week 12 (as defined by the Geboes, Robarts, and Nancy histopathology scores)
- Proportion of subjects with histologic improvement at Week 52 (as defined by the Geboes, Robarts, and Nancy histopathology scores)
- Proportion of subjects with histologic remission at Week 12 (as defined by the Geboes, Robarts, and Nancy histopathology scores)
- Proportion of subjects with histologic remission at Week 52 (as defined by the Geboes, Robarts, and Nancy histopathology scores)
- Time to loss of response, with loss of response defined by:
   - A ≥ 2-point increase from Week 12 in the combined SF + RB scores and combined SF + RB score of ≥ 4, on 2 consecutive visits (≥ 7 days apart), and
   - Confirmed by centrally read ES ≥ 2 and,
   - Confirmation of negative C. difficile testing
- Proportion of subjects with improvement in EIMs at Weeks 12 and 52, in subjects with EIMs at baseline
- Scores and change from baseline at Weeks 12 and 52 in the following:
   - IBDQ total score
   - UC-PRO/SS
   - SF-36, version 2, physical and mental component and domain scores
   - WPAI-UC
   - Urgency NRS
   - Abdominal pain NRS
- Proportion of subjects with UC-related hospitalizations
- Proportion of subjects requiring UC-related surgeries, including colectomy
- Plasma concentrations of Compound 1, M3, and other metabolite(s) of interest (if warranted) will be assessed from samples collected prior to dosing and 4 hours (± 15 minutes) post-dose (after 12-lead ECG) on Week 0/Day 1
- Plasma concentrations of Compound 1, M3, and other metabolite(s) of interest (if warranted) will be assessed from samples collected prior to dosing (trough) at Weeks 2, 4, 8, 12, 16, 20, 24, 32, 40, 48, and 52
- Change from baseline in level of fecal calprotectin at Weeks 4, 8, 12, 24, and 52
- Change from baseline in level of hs-CRP at Weeks 2, 4, 8, 12, 16, 20, 24, 32, 40, 48, and 52
- Change and percentage change from baseline in lymphocyte counts at Weeks 2, 4, 8, 12, 16, 20, 24, 32, 40, 48, and 52
- Incidence and severity of laboratory abnormalities, and change from baseline in laboratory values (to include hematology, serum chemistry, coagulation, and urinalysis)
- Incidence of clinically significant vital sign abnormalities and changes from baseline

Evaluations will be performed at weeks 2, 4, 8, 12, 16, 20, 24, 32, 40, 48, and 52. Eligible participants will be given the opportunity to enter an open label extension study (OLE) with Compound 1.

## Claims

1. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof,
for use in a method of treating a sphingosine 1-phosphate subtype 1 (S1P₁) receptor-associated disorder in an individual in need thereof,
wherein the S1P₁ receptor-associated disorder is inflammatory bowel disease,
the method comprising the steps of:
selecting an individual who has not been previously treated with a therapeutically effective amount of an integrin receptor antagonist; and
administering to the individual a standard dose of Compound 1 or pharmaceutically salt, solvate, or hydrate thereof, in an amount equivalent to about 0.5 to about 5.0 mg of Compound 1.

2. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to claim 1,
wherein the integrin receptor antagonist is vedolizumab.

3. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof,
for use in a method of treating a sphingosine 1-phosphate subtype 1 (S1P₁) receptor-associated disorder in an individual in need thereof,
wherein the S1P₁ receptor-associated disorder is inflammatory bowel disease,
the method comprising the steps of:
selecting an individual who does not have a history of pancolitis; and
administering to the individual a standard dose of Compound 1 or pharmaceutically salt, solvate, or hydrate thereof, in an amount equivalent to about 0.5 to about 5.0 mg of Compound 1.

4. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to claim 3,
wherein the individual with a history of pancolitis had pancolitis for about, or greater than about, 1, 2, 3, 4, 5, 6, 7, 8, 9, or 10 years.

5. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-4,
wherein the treating comprises:
inducing and/or maintaining clinical response;
improving endoscopic appearance of the mucosa; and/or
inducing and/or maintaining clinical remission.

6. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-5,
wherein the standard dose is administered without titration.

7. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-6,
wherein the inflammatory bowel disease is ulcerative colitis;
for example, moderately to severely active ulcerative colitis.

8. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-6,
wherein the inflammatory bowel disease is Crohn's disease.

9. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-8,
wherein the standard dose is in an amount equivalent to 1 mg of Compound 1.

10. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-8,
wherein the standard dose is in an amount equivalent to 2 mg of Compound 1.

11. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-8,
wherein the standard dose is in an amount equivalent to 3 mg of Compound 1.

12. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-11,
wherein the standard dose of Compound 1, or a pharmaceutically acceptable salt, hydrate, or solvate thereof is administered once daily to the individual.

13. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-12,
wherein the Compound 1, or a pharmaceutically acceptable salt, hydrate, or solvate thereof, is selected from:
Compound 1;
a calcium salt of Compound 1; and
an L-arginine salt of Compound 1.

14. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-12,
wherein the Compound 1, or a pharmaceutically acceptable salt, hydrate, or solvate thereof, is an L-arginine salt of Compound 1.

15. (*R*)-2-(7-(4-cyclopentyl-3-(trifluoromethyl)benzyloxy)-1,2,3,4-tetrahydrocyclo-penta[b]indol-3-yl)acetic acid (Compound 1) or a pharmaceutically salt, solvate, or hydrate thereof, for use according to any one of claims 1-12,
wherein the Compound 1, or a pharmaceutically acceptable salt, hydrate, or solvate thereof, is an anhydrous, non-solvated crystalline form of an L-arginine salt of Compound 1.

## Patentansprüche

1. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon
zur Verwendung in einem Verfahren zur Behandlung einer Sphingosin-1-phosphat-Subtyp-1 (S1P₁) -Rezeptor-assoziierten Störung bei einem dafür bedürftigen Individuum,
wobei die S1P₁-Rezeptor-assoziierte Störung eine entzündliche Darmerkrankung (inflammatory bowel disease) ist,
wobei das Verfahren die Schritte umfasst aus:
Auswählen eines Individuums, das zuvor nicht mit einer therapeutisch wirksamen Menge eines Integrinrezeptorantagonisten behandelt wurde; und
Verabreichen einer Standarddosis von Verbindung 1 oder eines pharmazeutischen Salzes, Solvats oder Hydrats davon an das Individuum in einer Menge, die etwa 0,5 bis etwa 5,0 mg der Verbindung 1 entspricht.

2. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach Anspruch 1,
wobei der Integrinrezeptorantagonist Vedolizumab ist.

3. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon
zur Verwendung in einem Verfahren zur Behandlung einer Sphingosin-1-phosphat-Subtyp-1 (S1P₁) -Rezeptor-assoziierten Störung bei einem dafür bedürftigen Individuum,
wobei die S1P₁-Rezeptor-assoziierte Störung eine entzündliche Darmerkrankung (inflammatory bowel disease) ist,
wobei das Verfahren die Schritte umfasst aus:
Auswählen eines Individuums, das keine Vorgeschichte von Pancolitis aufweist; und
Verabreichen einer Standarddosis von Verbindung 1 oder eines pharmazeutischen Salzes, Solvats oder Hydrats davon an das Individuum in einer Menge, die etwa 0,5 bis etwa 5,0 mg der Verbindung 1 entspricht.

4. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach Anspruch 3,
wobei das Individuum mit einer Vorgeschichte von Pancolitis etwa oder mehr als etwa 1, 2, 3, 4, 5, 6, 7, 8, 9 oder 10 Jahre lang Pancolitis hatte.

5. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-4,
wobei die Behandlung umfasst:
Induzieren und/oder Aufrechterhalten von klinischem Ansprechen;
Verbessern eines endoskopischen Erscheinungsbildes der Schleimhaut; und/oder
Induzieren und/oder Aufrechterhalten von klinischer Remission.

6. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-5,
wobei die Standarddosis ohne Titration verabreicht wird.

7. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-6,
wobei die entzündliche Darmerkrankung Colitis ulcerosa ist;
zum Beispiel mäßig bis schwer aktive Colitis ulcerosa.

8. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-6,
wobei die entzündliche Darmerkrankung Morbus Crohn ist.

9. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-8,
wobei die Standarddosis eine Menge ist, die 1 mg der Verbindung 1 entspricht.

10. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-8,
wobei die Standarddosis eine Menge ist, die 2 mg der Verbindung 1 entspricht.

11. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-8,
wobei die Standarddosis eine Menge ist, die 3 mg der Verbindung 1 entspricht.

12. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-11,
wobei die Standarddosis von Verbindung 1 oder eines pharmazeutisch annehmbaren Salzes, Hydrats oder Solvats davon einmal täglich an das Individuum verabreicht wird.

13. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-12,
wobei die Verbindung 1 oder ein pharmazeutisch annehmbares Salz, Hydrat oder Solvat davon ausgewählt ist aus:
Verbindung 1;
einem Calciumsalz von Verbindung 1; und
einem L-Argininsalz von Verbindung 1.

14. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-12,
wobei die Verbindung 1 oder ein pharmazeutisch annehmbares Salz, Hydrat oder Solvat davon ein L-Argininsalz von Verbindung 1 ist.

15. (*R*)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisches Salz, Solvat oder Hydrat davon zur Verwendung nach einem der Ansprüche 1-12,
wobei die Verbindung 1 oder ein pharmazeutisch annehmbares Salz, Hydrat oder Solvat davon eine wasserfreie, nicht solvatisierte kristalline Form eines L-Argininsalzes der Verbindung 1 ist.

## Revendications

1. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci,
pour une utilisation dans un procédé de traitement d'un trouble associé au récepteur de la sphingosine 1-phosphate de sous-type 1 (S1P₁) chez un individu qui en a besoin,
dans lequel le trouble associé au récepteur S1P₁ est une maladie intestinale inflammatoire,
le procédé comprenant les étapes suivantes :
sélectionner un individu qui n'a pas été précédemment traité avec une quantité thérapeutiquement efficace d'un antagoniste de récepteur d'intégrine ; et
administrer à l'individu une dose standard de Composé 1 ou un sel, solvate ou hydrate pharmaceutique de celui-ci, en une quantité équivalente à environ 0,5 à environ 5,0 mg de Composé 1.

2. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon la revendication 1,
dans lequel l'antagoniste de récepteur d'intégrine est le védolizumab.

3. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci,
pour une utilisation dans un procédé de traitement d'un trouble associé au récepteur de la sphingosine 1-phosphate de sous-type 1 (S1P₁) chez un individu qui en a besoin,
dans lequel le trouble associé au récepteur S1P₁ est une maladie intestinale inflammatoire,
le procédé comprenant les étapes consistant à
sélectionner un individu qui n'a pas d'antécédents de pancolite ; et
administrer à l'individu une dose standard de Composé 1 ou un sel, solvate ou hydrate pharmaceutique de celui-ci, en une quantité équivalente à environ 0,5 à environ 5,0 mg de Composé 1.

4. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon la revendication 3,
dans lequel l'individu ayant des antécédents de pancolite a eu une pancolite pendant environ, ou plus d'environ, 1, 2, 3, 4, 5, 6, 7, 8, 9 ou 10 ans.

5. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 4,
dans lequel le traitement comprend :
l'induction et/ou le maintien d'une réponse clinique ;
l'amélioration de l'aspect endoscopique de la muqueuse ; et/ou
l'induction et/ou le maintien d'une rémission clinique.

6. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 5,
dans lequel la dose standard est administrée sans titrage.

7. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 6,
dans lequel la maladie intestinale inflammatoire est une colite ulcéreuse ;
par exemple, une colite ulcéreuse modérément à sévèrement active.

8. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 6,
dans lequel la maladie intestinale inflammatoire est la maladie de Crohn.

9. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 8,
dans lequel la dose standard est en une quantité équivalente à 1 mg de Composé 1.

10. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 8,
dans lequel la dose standard est en une quantité équivalente à 2 mg de Composé 1.

11. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 8,
dans lequel la dose standard est en une quantité équivalente à 3 mg de Composé 1.

12. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 11,
dans lequel la dose standard de Composé 1, ou d'un sel, hydrate ou solvate pharmaceutiquement acceptable de celui-ci est administrée une fois par jour à l'individu.

13. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 12,
dans lequel le Composé 1, ou un sel, hydrate ou solvate pharmaceutiquement acceptable de celui-ci, est sélectionné parmi :
Composé 1 ;
un sel de calcium de Composé 1 ; et
un sel de L-arginine de Composé 1.

14. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 12,
dans lequel le Composé 1, ou un sel, hydrate ou solvate pharmaceutiquement acceptable de celui-ci, est un sel de L-arginine de Composé 1.

15. Acide (R)-2-(7-(4-cyclopentyl-3-(trifluorométhyl)benzyloxy)-1,2,3,4-tétrahydrocyclo-penta[b]indol-3-yl)acétique (Composé 1) ou sel, solvate ou hydrate pharmaceutique de celui-ci, pour une utilisation selon l'une quelconque des revendications 1 à 12,
dans lequel le Composé 1, ou un sel, hydrate ou solvate pharmaceutiquement acceptable de celui-ci, est une forme cristalline anhydre non solvatée d'un sel de L-arginine de Composé 1.
